# EUROPEAN PATENT APPLICATION

(11) **EP 3 560 461 A1**
(43) Date of publication of application: **30.10.2019**
(21) Application number: 19171687.7
(22) Date of filing: 29.04.2019
(51) Int. Cl.: A61F 2/958

(54) **COMBINATION DELIVERY SYSTEM**

(30) Priority: 27.04.2018 US 201862663417 P
(71) Applicant: Cook Medical Technologies, LLC, Bloomington, IN 47404 (US)
(72) Inventor: MAYLE, Brent A., Spencer, IN 47460 (US); PAUL, Jr., Ram H., Bloomington, IN 47403 (US); MERK, James C., Terre Haute, IN 47802 (US); NEFF, Gary, Bloomington, IN 47408 (US); PUCKETT, Dean R., Bloomington, IN 47403 (US); NEIDIGH, Austin, Bloomfield, IN 47424 (US); MINDEL, Thomas, Bloomington, IN 47404 (US)
(74) Representative: Leach, Sean Adam

(57) **Abstract**

Disclosed herein is an assembly including a balloon catheter as an inner catheter for the delivery of a self-expanding stent. The delivery assembly may lack a separate pusher structure. A method of using a single assembly to delivery a self-expanding stent and provide a post-dilatation step is also described herein.

## Description

### BACKGROUND

The present application generally relates to medical devices. More particularly, the present application relates to stents and delivery systems for stents.

Stents of different construction have been used to treat a variety of conditions. When stiffness is desired, a balloon-expandable (BX) stent is sometimes selected, as its shape can be set during deployment by application of an inflation force from a balloon of the delivery system. In applications for treatment more tortuous anatomy, a self-expanding (SX) stent may be desired for its relative flexibility.

Hybrid stents combining balloon-expandable and self-expanding sections have been contemplated in the art, but none are available in the market owing to the fact that construction of a hybrid stent which performs as desired is a major challenge. First, the materials used to construct balloon-expandable and self-expanding sections are generally dissimilar, and conventional joining methods are likely to fail. Second, during deployment, a stent is subjected to various forces from the delivery system. The forces experienced by a balloon-expandable stent are different from those experienced by an self-expanding stent, which can endanger the survival of the joint between the two sections, assuming the two can be joined in the first place.

Certain medical implants include balloon-expandable and self-expanding stents in a single device. Among these are fenestrated stent grafts, in which a balloon-expandable stent is disposed through the fenestration of the main body of the stent graft, and an self-expanding stent extends as the body of a side branch, extending away from the main body into a branch vessel. In such an instance, the self-expanding stent is joined to the balloon-expandable stent via graft material.

Because of the varied forces that need to be applied to and withstood by the device, it may be beneficial to develop delivery systems which are capable of delivering a hybrid stent having a balloon-expandable portion and an self-expanding portion. Such a device would allow for the expansion of the self-expanding portion while providing a balloon for expansion of the balloon-expandable portion. Additionally, such a device may be useful in the delivery of standard self-expanding stents, as current methods involve delivery and implantation with a first system which carries the device within the vasculature, the withdrawal of that delivery system, and the provision of a second system to the implantation site, which carries a balloon to affect a dilatation step in which the implant is patent with the vessel wall.

It has been a challenge to develop a hybrid stent which has a balloon-expandable portion and an self-expanding portion which can withstand delivery intact, and a delivery system capable of delivering such a stent. It has likewise been a challenge to provide a single delivery system and delivery method capable of delivering an self-expanding stent and dilating the self-expanding stent such that it is seated patent against the wall of the body vessel to which it has been delivered.

### SUMMARY

In one aspect, the present disclosure provides a stent. The stent includes a tubular body which extends from a first end to a second end and defines a lumen therethrough. The tubular body may include a first portion extending from the first end to a third end. The first portion may be a self-expanding tubular body. The stent may include a second portion extending from the second end to a fourth end. The second portion may be a balloon-expandable tubular body. The stent may include an attachment selected from one of a rivet and a solder joint comprising at least two solders, the attachment joining the first portion to the second portion. The attachment may be capable of withstanding a total shearing force of about 20 newtons (N) applied during deployment from a delivery system, or a shearing force of about 0.1 N to about 0.5 N at each joint between the balloon-expandable portion and the self-expanding portion.

In another aspect, the present disclosure provides a method of making a stent. The method includes disposing a first solder comprising a first flux onto an end of a self-expanding tubular body to form a first coated portion. The method includes disposing a second solder comprising a second flux onto an end of a balloon-expandable tubular body to form a second coated portion. The second solder and the second flux are distinct from the first solder and the first flux, respectively. The method may include contacting the first coated portion with the second coated portion. The method may further include heating the first coated portion and the second coated portion to join the self-expanding tubular body to the balloon-expandable tubular body, thereby forming the stent.

In another aspect, the present disclosure provides a medical device assembly. The medical device assembly includes a balloon catheter having a catheter body extending from a proximal end to a distal end and defining a lumen therethrough, and at least one inflatable balloon disposed circumferentially about a portion of the catheter body. The at least one inflatable balloon has an interior in fluid communication with the lumen of the catheter body. The medical device assembly includes a stent which may have a tubular body extending from a first end to a second end and defining a lumen therethrough. The tubular body may include a first portion extending from the first end to a third end. The first portion may be a self-expanding tubular body. The tubular body may include a second portion extending from the second end to a fourth end. The second portion may be a balloon-expandable tubular body. An attachment may join the first portion to the second portion. In the medical device assembly, at least the second portion of the stent being disposed over at least one inflatable balloon of the balloon catheter.

In another aspect, a medical device assembly is described. The medical device assembly may include an outer sheath and a balloon catheter disposed within the outer sheath. The balloon catheter may include a catheter body extending from a proximal end to a distal end and defining a lumen therethrough, and an inflatable balloon disposed circumferentially about a portion of the catheter body and extending from a first proximal end to a first distal end, the inflatable balloon having an interior in fluid communication with the lumen of the catheter body. The medical device assembly may include a self-expanding stent extending from a second proximal end to a second distal end and disposed about the inflatable balloon such that the first proximal end of the inflatable balloon contacts and at least partially abuts the second proximal end of the self-expanding stent.

In another aspect, the present disclosure provides a method of making a medical device assembly. The method may include loading a self-expanding stent into an outer sheath, the self-expanding stent extending from a stent proximal end to a stent distal end and having a first length, the self-expanding stent expanding to an inner diameter of the outer sheath after loading. The method also may include loading a balloon catheter into the outer sheath, the balloon catheter including an inflatable balloon extending from a balloon proximal end to a balloon distal end and having a second length greater than the first length, the inflatable balloon being positioned such that the balloon proximal end lies proximal of the stent proximal end and the balloon distal end lies distal of the stent distal end. The method may also include pressurizing the inflatable balloon such that the balloon proximal end contacts and at least partially abuts the stent proximal end.

In another aspect, the present disclosure provides a method of delivering a stent. The method may include delivering a medical device assembly in a compressed configuration to a site of treatment within a body lumen of a patient. The medical device assembly may include a balloon catheter. The balloon catheter may include an inflatable balloon, the inflatable balloon including a balloon proximal end and extending to a balloon distal end. The medical device assembly may include a self-expanding stent. The self-expanding stent may include a stent proximal end and extend to a stent distal end. The self-expanding stent may be disposed over the inflatable balloon such that the balloon proximal end at least partially abuts the stent proximal end such that the inflatable balloon reduces longitudinal movement of the stent during deployment. The medical device assembly may include an outer sheath surrounding the balloon catheter and self-expanding stent, such that the inflatable balloon acts to retain the stent against longitudinal movement during deployment. The method may include withdrawing the outer sheath proximally to deploy the self-expanding stent to the site of treatment. The method may include inflating the inflatable balloon of the balloon catheter to seat the self-expanding stent against a wall of the body lumen at the treatment site.

Further objects, features and advantages of this system will become readily apparent to persons skilled in the art after a review of the following description, with reference to the drawings and claims that are appended to and form a part of this specification.

### BRIEF DESCRIPTION OF THE DRAWINGS

Figure 1 is a plan view of a balloon-expandable stent portion and a self-expanding stent portion for construction of a hybrid stent in accordance with the principles of the present invention;
Figure 2 is a hybrid stent constructed from a balloon-expandable stent portion and a self-expanding stent portion;
Figure 3 illustrates steps in a method of construction of a hybrid stent in accordance with an aspect of the present disclosure;
Figure 4 is an embodiment of an interface of two stent portions in accordance with an aspect of the present disclosure;
Figure 5 is another embodiment of an interface between two stent portions in accordance with another aspect of the present disclosure;
Figure 6 is another embodiment of an interface between two stent portions in accordance with another aspect of the present disclosure;
Figure 7 is an embodiment of a hybrid stent having three sections in accordance with an aspect of the present disclosure;
Figure 8 is an end view of a stent graft constructed with a hybrid stent in accordance with an embodiment of the present disclosure;
Figure 9 is a side view of a portion of the stent graft of Figure 8;
Figure 10 is a cross-sectional view of an embodiment of a delivery system constructed in accordance with an embodiment of the present disclosure;
Figure 11 is a cross-sectional view of another embodiment of a delivery system constructed in accordance with an embodiment of the present disclosure;
Figure 12 is a cross-sectional view of another embodiment of a delivery system constructed in accordance with an embodiment of the present disclosure;
Figure 13 is a cross-sectional view of another embodiment of a delivery system constructed in accordance with an embodiment of the present disclosure;
Figure 14 is a cross-sectional view of another embodiment of a delivery system constructed in accordance with an embodiment of the present disclosure;
Figures 15A, 15B, 15C, and 15D are steps in the release of a hybrid stent from a sheath in accordance with an embodiment of the present disclosure;
Figures 16A, 16B, 16C, and 16D are steps in the delivery of a hybrid stent using a wedge element in accordance with another embodiment of the present disclosure;
Figure 17 illustrates a method of delivering a self-expanding stent over a balloon catheter in accordance with the principles of the present disclosure;
Figure 18 is a view of a self-expanding stent disposed over a balloon catheter for delivery in accordance with another embodiment of the present disclosure; and
Figure 19 is a side view of a handle assembly for use in accordance with another embodiment of the present disclosure.

### DETAILED DESCRIPTION

The drawings are purely schematic illustrations of various aspects of the invention and are not necessarily to scale, unless expressly stated.

The terms "substantially" or "about" used herein with reference to a quantity includes variations in the recited quantity that are equivalent to the quantity recited, such as an amount that is equivalent to the quantity recited for an intended purpose or function. "Substantially" or derivatives thereof will be understood to mean significantly or in large part.

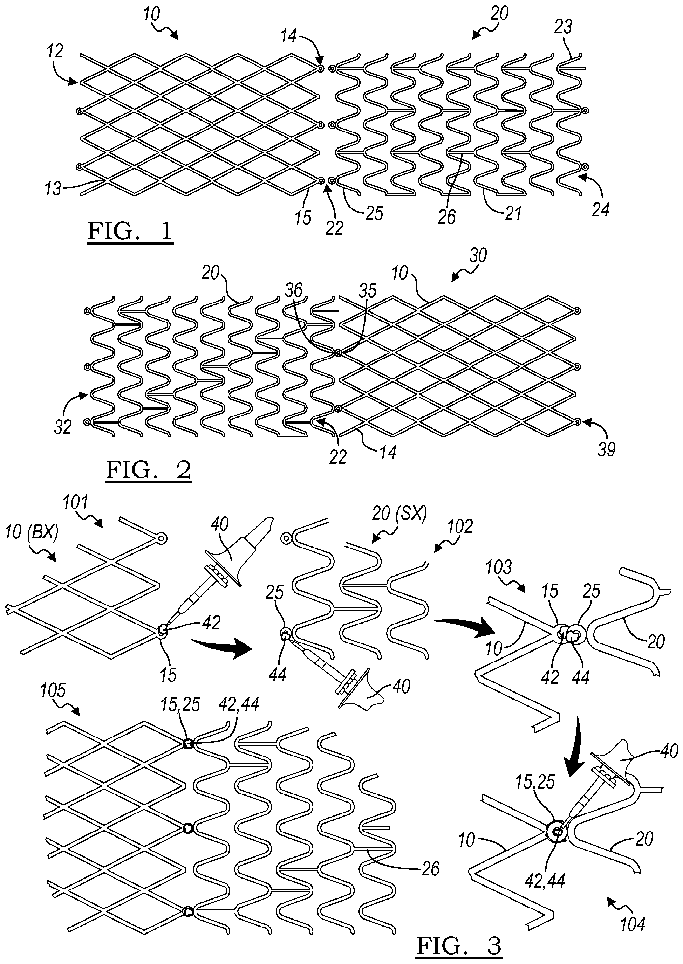

A hybrid stent may be made by joining a balloon-expandable stent to a self-expanding stent. As shown in Figure 1, a balloon-expandable stent portion 10 extends from first end 12 to third end 14 to define a stent portion body 13. Likewise, a self-expanding stent portion 20 extends from a second end 22 to a fourth end 24, to define a stent portion body 23 therebetween.

Numerous designs are known in the art for a variety of balloon expandable and self-expanding stents. Although any known design of a stent is contemplated as being compatible as a stent portion with the principles of the present disclosure, those balloon expandable and self-expanding stent portions which are cut from a precursor cannula are particularly suited for such a purpose. Materials appropriate for precursor cannulas will be known by those of skill in the art.

For example, in one embodiment, the balloon expandable stent portion may be cut from a stainless steel cannula or one made of a cobalt chromium alloy, and the self-expanding stent portion 20 may be made from a shape memory metal, such as a nickel titanium alloy.

As shown in Figure 1, the balloon expandable stent portion may have a closed cell structure. In other embodiments, the balloon-expandable stent portion may have a more open structure. In an embodiment, the self-expanding stent portion body 23 may be made up of a plurality of coaxial rings 25, which are in turn made up of a plurality of struts 21. The plurality of rings 25 may be connected via one or more connectors 26. As has been depicted in Figure 1, the rings may be connected by a plurality of connectors 26, and these connectors 26 may be aligned axially along the length of the device 20. Such an arrangement may increase the column strength of the self-expanding stent portion 20 relative to other arrangements, and may assist in reducing shortening of the stent during deployment.

However, in other embodiments, it will be suitable to have connectors 26 arranged in a different relationship; that is, out of axial alignment.

The balloon-expandable stent portion 10 can be joined to the self-expanding stent 20 to create a hybrid stent 30 as is shown in Figure 2. In this Figure, second end 14 of the balloon-expandable stent portion 10 has been joined to second end 22 of the self-expanding stent portion, thereby resulting in the formation of junction 36. The hybrid stent 30 extends from first hybrid stent end 32 to second hybrid stent end 39, which are concurrent with fourth end 24 of the self-expanding stent portion and first end 12 of the balloon expandable stent portion, respectively. As will be apparent to one of skill in the art, other end-to-end to joining methods are possible in the construction of a hybrid stent.

In order to be delivered to the body vessel to be treated and to be an effective treatment, the hybrid stent 30 will ideally remain intact during and after delivery. The development of a hybrid stent has been difficult for this reason. The nature of the delivery system and the delivery procedure means that a number of forces will be applied to the stent; a compressive force from the outer sheath of the delivery system, for example, and axial forces of elongation (wherein the hybrid stent is stretched longer than its natural length, and the halves may be pulled apart) and an axial shearing force (wherein the balloon-expandable and self-expanding portions of the hybrid stent are compressed axially toward one another.) This shearing force is capable of breaking connections at the joints if the means of joining the balloon-expandable portion to the self-expanding portion is not sufficiently strong.

In some embodiments, the hybrid stent 30 is capable of withstanding a total shearing force of about 20 newtons (N) about the circumference of the device at the junction between the self-expanding and balloon-expandable portion. In some embodiments, each joint (such as each individual solder joint, or each rivet) at the junction between the self-expanding portion and the balloon-expandable portion is capable of withstanding about 0.1N to about 0.5N of shearing force, which is a local shearing force. The shearing force may be about applied locally to a unit of 0.03 mm² to any given portion of the hybrid stent. The shearing force is a translation of forces from the overall compressive forces measured in the stent for deployment that each element (including the joints at the junction of the balloon-expandable and self-expanding portions) is exposed to due to resistance associated with sliding the sheath over the stent for deployment.

The diameters of the self-expanding stent portion and the balloon expandable stent portion are substantially equal to one another in one embodiment. In another embodiment, one of the diameters may be slightly smaller than the other. For example, a balloon-expandable stent portion may have a smaller diameter in the expanded state than does the self-expanding stent portion, and an end of the balloon-expandable stent portion may be placed within the lumen of the self-expanding stent portion, thereby providing a contact point for joining the two and forming the hybrid stent. Moreover, the balloon-expandable stent and the hybrid stent may have different lengths. For instance, a longer self-expanding portion can be employed to create a longer overall stent.

In certain embodiments, the balloon-expandable stent portion 10 may be manufactured with a plurality of first eyelets 15 at at least one end, and likewise the self-expanding stent portion 20 may be manufactured with a plurality of second eyelets 25. If eyelets are provided, these may provide a suitable component at which the balloon-expandable stent portion 10 may be joined to the self-expanding stent portion 20. One or both of the devices may have eyelets, or, in another embodiment, neither device may have a plurality of eyelets. In the hybrid stent 30 depicted in Figure 2, the first eyelets 15 are joined to second eyelets 25 to form a plurality of joined eyelets 35 at junction 36.

As mentioned previously, the balloon-expandable stent portion 10 may be made of a different material than the self-expanding stent portion 20. It can be challenging to join two dissimilar materials. In particular, if one of the two precursor stent portions is made of a shape memory alloy, it is known that these materials are difficult to manipulate, even without the additional complication of attempting to join to a dissimilar material.

One joining method is illustrated in Figure 3. In a first step 101, a balloon-expandable stent portion 10 having a joining portion, such as plurality of eyelets 15, is provided. A first flux 42 compatible with the material from which the balloon-expandable stent portion 10 is made is deposited on the joining portion (eyelet 15), such as by brushing or by dipping. Then, solder is applied over the first flux is and allowed to set. Alternatively, the first flux 42 may be provided in conjunction with a solder (such as in the core of a solder wire) and may be provided using a soldering iron 40, as illustrated in Figure 3.

Similarly, in step 102, a second flux 44 is applied to a joining portion of a self-expanding stent portion 20, in the illustrated case eyelets 25. The second flux 44 is then covered with solder. Therefore the material of the balloon-expandable stent portion 10 and of the self-expanding stent portion 20 are selected such that the first flux 42 and the second flux 44 are compatible with the materials of the two stent portions.

The first flux 42 may be used with a first solder, and the second flux 44 may be used with a second solder. The first solder may be different from the second solder, or the first solder may be the same as the second solder. The first flux 42 may be different from the second flux 44.

In a third step 103, the solder deposited on the balloon-expandable stent portion 10 is brought into contact with the solder deposited on the self-expanding stent portion 20.

In step 104, heat is applied to at least partially liquefy the solders of both stent portion. After a time, the heat is removed, and step 105 yields the hybrid stent 30 which is joined at the eyelets 15 and 25 by a single solder joint, making use of the two different fluxes.

In one example, the balloon expandable stent portion 10 may be made of stainless steel, and the self-expanding stent portion 20 may be made of a nickel-titanium alloy, such as NITINOL. A steel-compatible flux, such as STAY-CLEAN zinc chloride flux, may be used to cover a portion of the stainless steel stent portion, such as a stainless steel eyelet, and a titanium-compatible flux such as an acid-based solder not containing zinc or chlorine, and which can remove titanium and nickel oxides, may be used to cover a portion of the self-expanding stent portion, such as an eyelet. A solder compatible with both fluxes may then be used, such as ALLSTATE 430 soft silver solder. As mentioned, the solder and respective flux may be applied separately, or the solder and flux may be provided together.

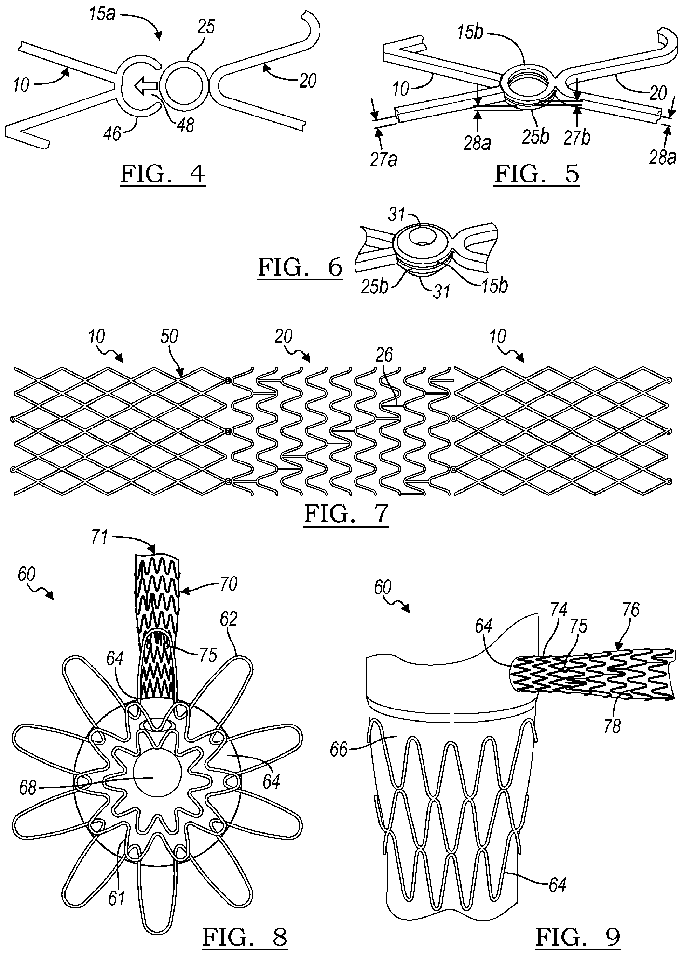

If applied to two stents with a standard thickness along their entire lengths, the method of Figure 3 will yield a hybrid stent 30 with a thickest portion at the junction 36. Therefore, a number of modifications to the eyelets are contemplated. As illustrated in Figure 4, at least one of the eyelets (in this case, first eyelet 15a of the balloon-expandable stent portion 10) may be slit such that the eyelet 15a can have an open configuration 46. In the embodiment illustrated in Figure 4, the slit is located on the circumference of the eyelet 15a at the extreme end of the device 10, but in other embodiments it may be formed anywhere on the eyelet 15a. The second eyelet 25 of the self-expanding stent portion 20 is a standard closed eyelet 25 which can be disposed such that it is circumferentially surrounded by the first eyelet 15a. The stents 10 and 20 can then be joined by any method to yield a hybrid stent 30.

In another embodiment, illustrated in Figure 5, both the balloon-expandable stent portion 10 and the self-expanding stent portion 20 may have modified joining portions; in the illustrated embodiment, first eyelets 15a and second eyelets 15b. In this embodiment, the struts of the balloon-expandable stent portion 10 have a first thickness 27a, and the eyelet (or junction portion, or joining portion) has a second thickness 27b which is less than the first thickness 27a. Likewise, the self-expanding stent portion 20 has struts of a third thickness 28a, and the second eyelets a fourth thickness 28b less than the third thickness 28a. As such, the eyelets are thinned compared to the remainder of the device. In an environment in which the stent portion 10 or 20 is cut from a precursor cannula, this thinning may be achieved after the stent has been cut from the cannula, such as by electropolishing, or by another thinning method. This allows the hybrid stent 30 resulting from the joining of eyelet 15b to eyelet 25b to have a substantially consistent thickness across its entire length.

The self-expanding stent portion 20 may be joined to the balloon-expandable stent portion 10 by a number of different mechanisms. As already described, one such method includes using a flux compatible with the material of each stent, and then reheating such that two solders flowed over the flux can be joined. In some embodiments, the solders may be applied to eyelets of the stent portions, and may flow into the eyelets, as though to form a rivet made of solder through both eyelets of both stent portions of the hybrid stent.

Figure 6 illustrates another type of connection that can be used to join a self-expanding stent portion 20 to a balloon-expandable stent portion 10 to manufacture a hybrid stent 30. In this environment, the eyelets are aligned in the radial dimension and a rivet 31 is disposed through both eyelets. The rivet 31 is then crimped down to join the balloon-expandable stent portion 10 to the self-expanding stent portion 20. The mechanical connection provided by a rivet 31 renders the junction 36 durable. A plurality of rivets 31 may be used to join some or all of the eyelets in such a paired fashion. In some embodiments, the rivet may be made of a pliable but radiopaque material, including but not limited to one of at least one of gold and platinum, in order to provide the device with a radiopaque marker for visualization during implantation, and monitoring of the implanted device.

Other methods may be used to attach the self-expanding and balloon-expandable portions of the hybrid stent, beyond soldering and rivets. For example, the two halves may be attached via a sonic weld, or a friction stir weld, or another joining method

A hybrid stent may provide better sizing to a vessel in which it is implanted than a stent which is only balloon-expandable or self-expanding. A hybrid stent may have a controllable radial force profile, may ease positioning of the implant, and may minimize or eliminate stent jumping during deployment. The hybrid stent design may minimize jumping and increase accuracy in positioning because one portion of the device is delivered while the other remains within the delivery system.

A hybrid stent as disclosed herein may be of particular assistance in bridging a relatively straight vessel (using a balloon-expandable portion as an anchor) to a more tortuous vessel (taking advantage of the self-expanding portion). Such hybrid stents may be constructed with varying amounts of covering along their length. In one embodiment, both the balloon-expandable portion and the self-expanding portion may be completely covered by graft material. In another embodiment, only portions the balloon-expandable and self-expanding portions may be covered. In another embodiment, the hybrid stent may be a bare metal stent. In one example, treatment of a calcified ostial lesion may benefit from a balloon-expandable portion to hold open the lesion, and the flexibility of the self-expanding portion may provide a transition to the healthy part of the vessel.

The present disclosure is not limited to hybrid stents derived from the joining of a single balloon-expandable stent portion 10 to a single self-expanding stent portion 20. As shown in Figure 7, a hybrid stent 50 may include more than two precursor stent portions. In the example illustrated in Figure 7, a self-expanding stent portion 20 is positioned between two balloon expandable stents 10. Such a device may be particularly useful in an area of anatomy in which two vessels to be supported are bridged by a curved vessel. A hybrid stent 50 as in Figure 7 could be used in a stent graft in a trauma application in which there is a transected vessel, wherein each balloon-expandable portion is positioned in a vessel end for securement of the device, and the central self-expanding portion bridges the two, allowing for a flexible central section.

Hybrid stents constructed in accordance with the principles of the present invention are useful for a number of applications. Turning now to Figures 8 and 9, a stent graft 60 is depicted. Stent graft 60 is a fenestrated stent graft, having a main body 61 which has at least one fenestration 64 formed through the graft material 66, which defines the tubular shape of the main body 61. The hybrid stent 72 in this embodiment is a structural component of side branch 70, which engages with main body 61 at and through fenestration 64. In one embodiment, the hybrid stent 72 is held in place by flaring of the balloon-expandable portion within the main body 61 of the stent graft 60. The side branch 70 has a side branch lumen 71 formed therethrough, which is in fluid communication with the main body lumen 68 via fenestration 64.

The two stent portions of the hybrid stent 72 of side branch 70 serve different purposes. balloon-expandable portion 74 serves as a flaring portion, imparting stiffness to the construct and interacting with the fenestration 64. The self-expanding portion 76 extends away from the main body 61 and allows for more flexibility and conformation to the natural curvature of vessel into which the side branch 70 is to be deployed.

Various procedures may dictate how much covering or graft material is used in a variety of portions of a stent graft in embodiments of the present disclosure. In some instances, the hybrid stent may be utilized as a bare metal stent. In other embodiments, the hybrid stent may be fully covered with a graft material. In still other embodiments, the hybrid stent may be partially covered, with covering over a portion of the stent and exposed metal at other portions along its length. The balloon-expandable and self-expanding stents making up such a hybrid stent for use in a stent graft may also be constructed at a variety of diameters, and may have a variety of coverings along their lengths.

The graft material 66 used in a stent graft 60 may be a tubular graft material, and may be non-porous so that it does not leak or sweat under physiologic forces. The graft material may be made of a biocompatible material, including but not limited to a DACRON® polyester, another polyester fabric, polytetrafluoroethylene (PTFE), expanded PTFE, THORALON®, a polyamide, and other synthetic materials known to those of skill in the art. Naturally occurring biomaterials, such as collagen, particularly a derived collagen material known as extracellular matrix (ECM), such as small intestinal submucosa (SIS), may also be employed. In some embodiments, the graft material may be constructed as a preshaped tube. In some embodiments, the graft material may be a woven material.

In some embodiments, a stent graft including a hybrid stent in accordance with the principles of the present disclosure may be constructed to have a smooth covering, such as one which is described in U.S. Patent Application No. 15/224,101, the entire contents of which are incorporated herein by reference.

A stent graft (or hybrid stent) constructed in accordance with the principles of the present invention may be suitable for number of applications. One such application is the transjugular intrahepatic portosystemic shunt (TIPS) procedure. In TIPS, an artificial channel within the liver is created to establish communication between the inflow portal vein and the outflow hepatic vein in order to treat, among other conditions, portal hypertension. This procedure is currently conducted with multiple devices, or a single non-hybrid stent. A hybrid stent or a stent graft including a hybrid stent may be delivered to the liver, in some embodiments over a wire guide, such that the distal end of the hybrid stent protruding from the liver and into portal vein would be a self-expanding portion, and the proximal section spanning the tract newly formed in the liver would be the balloon-expandable portion.

The use of a hybrid stent in a TIPS procedure may provide a number of advantages over present methodologies. First, the use of preselected precursor devices provides zoned diameter targets, and allow for a tunable gradient of pressure by selecting appropriate precursor stent portions from which to make the hybrid device. Moreover, the combination of a balloon-expandable portion and an self-expanding portion into a single hybrid device facilitates easy deployment of the hybrid device, while leaving a stiff device in place to shunt across the diseased liver. In addition, because the balloon-expandable portion can be manipulated by multiple balloon dilatations, the hybrid stent would have an adjustable character, allowing for a better fit within the anatomy.

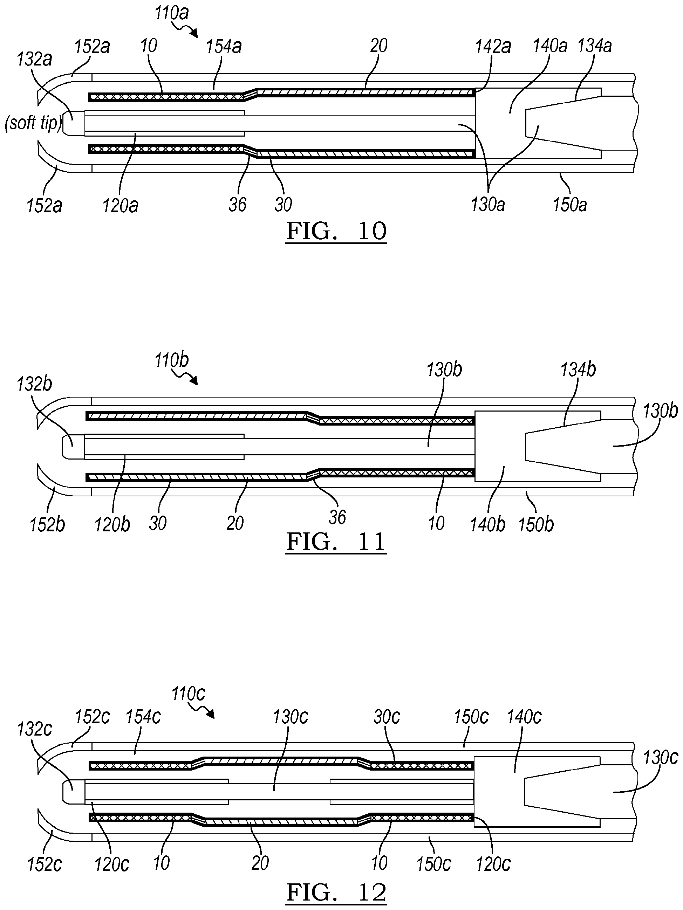

FIGs. 10-13 illustrate views of delivery systems which may be used to deliver and implant a hybrid stent as described herein. Such delivery systems may facilitate a multistep deployment scheme while making use of a single delivery system to be introduced to the body. A person of ordinary skill in the art will be aware that the dimensions of such a system may be tailored to the use of the particular device.

As mentioned, both balloon expandable and self-expanding stents are delivered to an implantation site in the lumen of a body vessel by specialized equipment which is tailored to the particular type of stent to be used. Figures 10 through 13 illustrate a number of embodiments of delivery systems which are suitable for delivering a hybrid stent that includes at least one self-expanding section and at least one balloon expandable section. A person of ordinary skill in the art will also appreciate that certain of these delivery systems may be suitable for delivering a self-expanding stent only, or a balloon expandable stent only.

Delivery system 110a, as shown in Figure 10, Includes an inner catheter 130a which is a balloon catheter. The hybrid stent 30 in this embodiment is disposed over the inner catheter 130a, with the balloon expandable portion 10 disposed or crimped over balloon 120a, and the self-expanding portion 20 compressed within outer sheath 150a over a section of inner catheter 130a which does not include the balloon 128. The balloon-expandable portion 10 may be crimped to a diameter such that it is in contact with the balloon 120a, or it may be slightly larger than this so that it does not contact the balloon, as illustrated in Figure 10. The junction 36 is the portion of hybrid stent 30 which lies between the balloon-expandable stent portion 10 and the self-expanding stent portion 20. The outer sheath 150a may optionally have at its distal end a flexible section 152a, which provides a delivery configuration in which the delivery system 110a is more compact, but also allows for straightforward ejection of the implant (hybrid stent 30) when the delivery system reaches the treatment site.

The inner catheter 130a likewise has at its distal end a soft tip 132a. At least one of soft tip 132a and flexible section 152a may further be embedded with radiopaque particles, or made of a radiopaque polymer, such that the delivery system may be tracked through the vasculature by known visualization methods, such as fluoroscopy. In the illustrated embodiment, the inner catheter 130a may have a smaller diameter in a distal section over which the stent is to be compressed and delivered, and may have a larger diameter in a portion proximal of this.

As shown in Figure 10, the inner catheter 130a may include a taper 134a as its diameter narrows to the lower profile toward the distal end. Optionally, a pusher element 140 may be provided with, or provided surrounding, the inner catheter 140a. The pusher element 140a, in the illustrated embodiment, provides a contact face 142a, which is in contact with the second end 39 of the hybrid stent 30. In the illustrated embodiment, the second end 39 is an end of the self-expanding stent portion 20.

In other embodiments, the delivery system 110a may not include a pusher element 140, as the crimped down section of the balloon-expandable stent portion 10 may provide sufficient anchoring such that the implant does not slide when the inner catheter 130a or the outer sheath 150a is moved, thereby further allowing for a decrease in profile of the delivery assembly 110a.

Figure 11 illustrates a delivery assembly 110b, which is another delivery assembly which can be used in accordance with the principles of the present disclosure. In this embodiment, the self-expanding portion of the hybrid stent 30 exits the delivery sheet 150b first, in contrast to the embodiment illustrated in Figure 10, in which the balloon expandable portion of the hybrid stent 30 exits the delivery system 110a first. In this embodiment, the components of the outer sheath 150b and the inner catheter 130b are largely arranged as they are in the embodiment of Figure 10, with the balloon being positioned distally and adjacent the distal tip 132b, and the self-expanding stent portion 20 of the hybrid stent 30 being effectively delivered over the balloon of the balloon catheter.

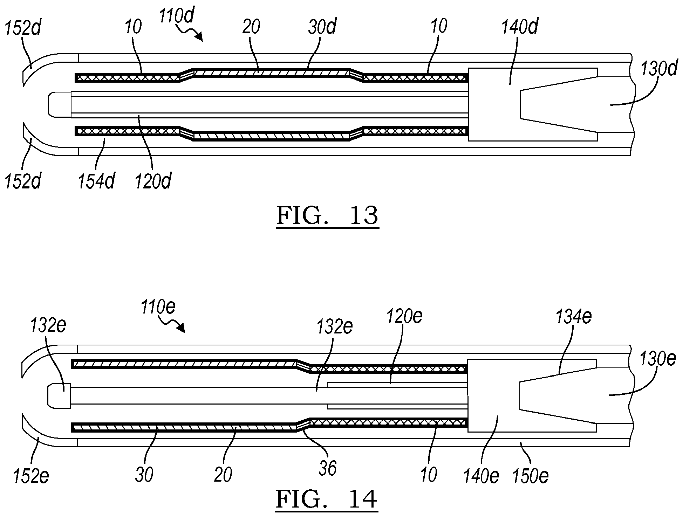

In another related embodiment, shown in Figure 14, the positioning of the balloon 120e may be changed, such that the balloon expandable portion 10 is delivered over the balloon 120e.

In some embodiments, the balloon-expandable portion 10 may be crimped over the balloon 120b/120e. In embodiments where the balloon expandable portion 10 is crimped over the balloon 120b/120e, the delivery assembly 110b/110e may be provided without a pusher band 140b/140e, as the crimped balloon-expandable stent portion 10 may provide axial stability for the device to be delivered within the delivery assembly 110b/110e.

In Figure 12, another embodiment of a delivery assembly 110c is illustrated. In this embodiment, the hybrid stent 130c includes three portions: two balloon expandable stents 10 at the proximal and distal ends of the hybrid stent 30C, and a self-expanding stent portion 20 bridging the two balloon expandable stent portions 10. In this embodiment, the inner catheter 130c is provided with two balloons 120c. The two balloon expandable portions 10 are each disposed over one of the balloons 120c. As in the foregoing examples if the balloon-expandable portion is disposed over a balloon in the delivery configuration, the delivery assembly may be provided without pusher 140c. In one embodiment, each of the balloons is provided with a separate inflation lumen for the delivery of inflation fluid; in another embodiment, the two balloons may share a common inflation lumen. A number of variations on this design are possible. For example, the two balloons maybe sized differently in the case that the diameters the final diameter of the different from one another in order to facilitate a size match with the anatomy, or to compensate for varying vessel size.

In some embodiments, a multiballoon delivery system may have two different expanded diameters. For instance, in one example the balloon-expandable portion of the hybrid stent may be an 8 millimeter (mm) diameter stent, and may be crimped over a 3 mm balloon. In this case, the 3 mm balloon may be inflated to slightly expand the balloon-expandable portion, and the delivery system may also provide an 8 mm balloon. After the initial expansion, the 8 mm balloon may be moved within the balloon-expandable portion of the stent and fully expanded to deploy the hybrid device. If a hybrid stent has multiple balloon-expandable portions, a delivery system of this design may have multiple 3 mm balloons, corresponding to the number of balloon-expandable portions.

Alternatively, having a balloon expandable segment 10 at each end and a self-expanding segment bridging the middle of the device may be delivered with the delivery system 120d as depicted in Figure 13. In this embodiment, rather than two small balloons as in Figure 12, a single balloon running the length of the entire implant is provided. As above, the balloon combined with the crimped balloon expandable stents could take the place of, or work in accordance with, a pusher band 140d.

In an alternative to the embodiments in which multiple balloons are employed, a single balloon having a plurality of different folded and/or expanded diameters along its length may instead be substituted, thereby offering the ability to have differing final diameters of the crimped and/or expanded stent portions associated with these portions. These teachings may be applied to the embodiments illustrated in FIGs. 10-14.

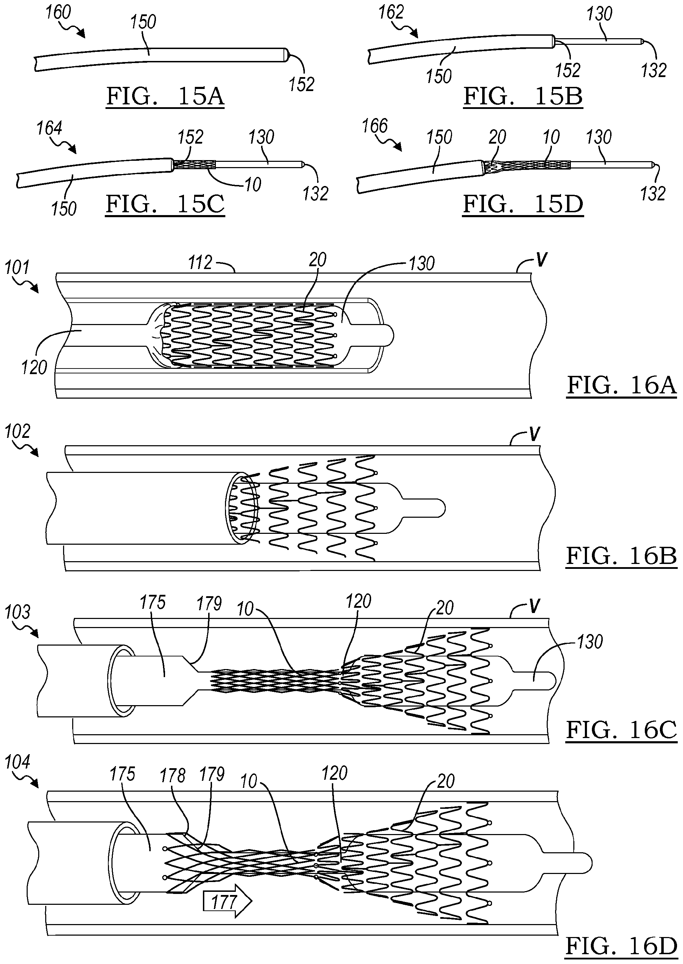

Figures 15A through 15D and 16A through 16D illustrate steps in the delivery of a hybrid stent in accordance with another embodiment of the present disclosure. These figures are directed to embodiments of delivery systems which offer a decreased circumferential profile for delivery to smaller vessels. In each of these embodiments, the balloon expandable portion 10 may be crimped over the catheter at a position that is not over a balloon. In one embodiment, the balloon expandable portion may be crimped distal the balloon, as shown in Figures 15A to 15D; in another embodiment, the balloon-expandable portion may be crimped proximal the balloon as shown in Figures 16A through 16D.

In the embodiment illustrated in Figures 15A through 15D, the balloon expandable portion 10 of hybrid stent 30 may be crimped distal of the balloon 120. Provided in the assembly and disposed over the inner catheter 130 is tapered sleeve 170, which has at its distal end a taper 174. This taper 174 may take the shape of a frustoconical section at the distal end of the sleeve 170, with the smallest diameter of the taper 174 being at the distal end of the sleeve 170 and the diameter increasing proximally. This taper 174 may function as a wedge, when the sleeve 170, which is disposed over the inner catheter 130, is moved distally through the interior of the balloon expandable portion 10, thereby slightly expanding the diameter of the balloon expandable portion 10 from its crimped condition. This then allows for relative movement of the inner catheter so that the balloon 120 may expand the balloon expandable portion 10 at the site of treatment, when the balloon 120 is inflated.

In one delivery method with a wedge element, and in which the balloon-expandable portion is deployed to the vessel first, the self-expanding portion may be contained within the sheath 150 to act as an anchor to prevent the entire hybrid stent from moving out of the proper location for deployment. The outer sheath can then be retracted in steps in which the balloon-expandable portion can be exposed one to two segments or cells at a time, and the inner balloon catheter and the wedge (in one embodiment, a 3 mm wedge) located proximal to the balloon can be pulled proximally into the exposed cells. This can be repeated 1-2 cells at a time until the balloon-expandable portion is completely exposed and slightly expanded. Following this, an 8 mm balloon positioned proximal of the wedge could be placed into the slightly-expanded stent for inflation to 8 mm. This process can be repeated for each balloon-expandable portion of a hybrid stent having multiple balloon-expandable portions. One of skill in the art will appreciate that the dimensions of a delivery system and a device as described herein can vary based on the application.

Likewise, in the embodiment illustrated in Figures 16A through 16D, the balloon expandable portion 10 may be crimped proximal of the balloon 120. In this embodiment, the self-expanding portion may be positioned over the balloon 120 of the inner catheter 130. In a similar way, tapered sleeve 175 is disposed over, and axially movable relative to, inner catheter 130. The tapered sleeve 175 includes a taper 179 which, when driven through the interior of the balloon expandable portion 10 by movement 177, expands to a partially expanded configuration 178. As in the examples of Figures 15A-D, the balloon may then be positioned within the interior of the balloon expandable portion, and inflated.

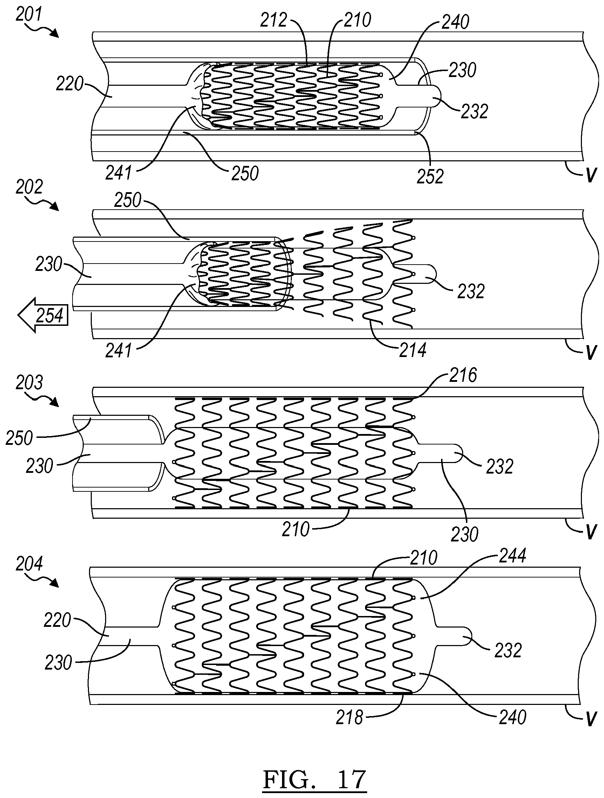

As mentioned above, with relation to the use of a pusher element in a delivery system for a hybrid stent, delivery of a self-expanding stent with a balloon catheter may be achieved. Figure 17 illustrates a scheme in which a single self-expanding stent 210 may be delivered over a balloon catheter 220 even in the absence of a crimped balloon expandable portion of a hybrid stent. That is, the self-expanding stent is entirely self-expanding from its first end to its second end, and has no balloon-expandable component to it. For example, the self-expanding stent may be cut from a single, monolithic cannula of a shape memory material.

Self-expanding stents are not typically deployed over a balloon catheter, as in general a pusher element is useful to provide resistance against the tendency of the self-expanding stent to track proximally as the outer sheath which constrains the self-expanding stent in its delivery configuration is withdrawn. However, in many procedures involving the delivery of a self-expanding stent, a balloon catheter is used after the self-expanding stent has been deployed. When the self-expanding stent has attained its expanded configuration at the desired location, the delivery system, except in some cases the wire guide over which the delivery system components are delivered, is withdrawn from the body of the patient, and a new system including a balloon catheter is introduced. The inflatable balloon of the balloon catheter is then disposed within the interior of the self-expanding stent, and a post-dilatation step is affected by the inflation of the balloon of the balloon catheter. Because this workflow involves introduction of multiple delivery systems, and because during delivery and withdrawal of a delivery system, wire guides need to be managed by the physician, eliminating the need for a second delivery system to permit for post-dilatation is desirable.

A method of delivery in which a single assembly can be used for delivery and post dilatation of a self-expanding stent is described in this embodiment. A self-expanding stent 210 is delivered over the balloon 240 of a balloon catheter 220, which has a catheter body 230 which runs through the balloon 240.

Step 201 in Figure 17 shows the self-expanding stent 210 in its compressed configuration 212 with balloon 240 within outer sheath 250. The assembly is introduced into vessel V. In this embodiment, the balloon 240 has a folded proximal end 241, which is disposed radially between the self-expanding stent 210 and the inner wall of the outer sheath 250. This fold at proximal end 241 acts as a securement, and functions as a pusher element, maintaining the position of the proximal end of the self-expanding stent 210 during delivery.

In step 202, the outer sheath 250 is withdrawn proximally 254. The distal end of self-expanding stent 210 exits the distal end 252 of the outer sheath 250. The distal end of the self-expanding stent 210 then expands and contacts the inner wall of the vessel V. The folded proximal end 241 of the balloon 240 is still disposed about the proximal end of the self-expanding stent 210. This gives rise to partially expanded configuration 214 of the self-expanding stent 210.

When the outer sheath 250 is fully withdrawn, the entire self-expanding stent 210 is delivered to the vessel, and the self-expanding stent 210 adopts its fully expanded configuration 216. The balloon 240 is still in a deflated state but is positioned within the interior of the self-expanding stent 210.

Finally, in step 204, fluid is introduced through an inflation lumen to inflate the balloon 240 which adopts its inflated configuration 244. This then further dilates the self-expanding stent 210 such that it is seated against the inner wall of the vessel consistently along its length. This leads to the generation of fully expanded and placed configuration 218 of the self-expanding stent 210. The balloon may then be deflated, and the delivery assembly withdrawn.

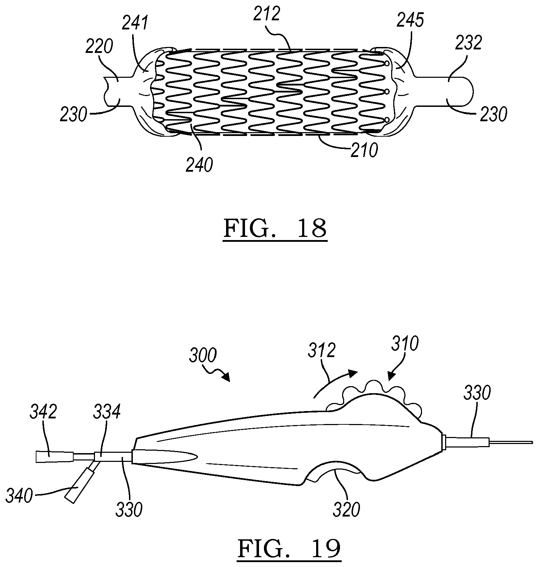

Figure 18 shows a variation on a compressed configuration 212 of a self-expanding stent for delivery in accordance with an embodiment similar to that shown in Figure 16. In this embodiment, not only does balloon 240 have a folded proximal end 241 radially disposed over the proximal end of self-expanding stent 210, but the distal end of balloon 240 adopts a folded distal end configuration 245 to provide an extra securement for the self-expanding stent 210. Such a configuration permits for the axial movement of the inner catheter 230 relative to the outer sheath 250 without permitting the self-expanding stent 210 to become axially displaced from the balloon 240.

A delivery assembly in accordance with the principles of the present disclosure may be equipped with a handle 300 at its proximal end as shown in Figure 19. The handle 300 includes a thumbwheel 310 which, when rotated clockwise 312, causes the inner catheter 330 to which it is attached to be advanced distally. When rotated counter-clockwise, the thumbwheel 310 allows for proximal retraction of the inner catheter 330. Switch 320 may function as a locking mechanism to hold the inner catheter in place. Alternatively, the thumbwheel 310 may move the outer sheath relative to the inner catheter 330, which stays in place, the inner catheter 330 being locked at the position at the position where it exits the handle 330. On the end of the handle 300 are ports 340 and 342 for the introduction of various components, such as for the delivery of fluid through the delivery assembly. To the extent that the methods described herein may be applied to tissues of the human or animal body, it will be appreciated that such methods may not provide any surgical or therapeutic effect. For example, such methods may be applied ex vivo, to tissue samples that are not part of the living human or animal body. In addition, the methods described herein may be practiced on meat, tissue samples, cadavers, and other non-living objects. Their implementation on cadavers may find particular utility in the training of surgeons and medical professionals. Certain methods may also be practiced on training dummies such as a non-living model of the living human or animal body. It will thus be appreciated that the methods described herein may not comprise a method of treatment of the living human or animal body by surgery or therapy.

As a person skilled in the art will readily appreciate, the above description is only meant as an illustration of implementation of the principles this application. This description is not intended to limit the scope of this application in that the system is susceptible to modification, variation and change, without departing from the spirit of this application, as defined in the following claims.

## Claims

1. A medical device assembly comprising:
an outer sheath;
a balloon catheter disposed within the outer sheath, the balloon catheter comprising:
a catheter body extending from a proximal end to a distal end and defining a lumen therethrough, and
an inflatable balloon disposed circumferentially about a portion of the catheter body and extending from a first proximal end to a first distal end, the inflatable balloon having an interior in fluid communication with the lumen of the catheter body; and
a self-expanding stent extending from a second proximal end to a second distal end and disposed about the inflatable balloon such that the first proximal end of the inflatable balloon contacts and at least partially abuts the second proximal end of the self-expanding stent.

2. The medical device assembly of claim 1, wherein the first distal end of the inflatable balloon contacts and at least partially abuts the second distal end of the self-expanding stent.

3. The medical device assembly of any preceding claim, wherein the self-expanding stent has a first length and the inflatable balloon has a second length greater than the first length.

4. The medical device assembly of any preceding claim, wherein the proximal end of the inflatable balloon is in contact with an inner wall of the outer sheath proximally of the proximal end of the stent.

5. The medical device assembly of any preceding claim, wherein the self-expanding stent comprises a plurality of apertures, for example wherein the inflatable balloon protrudes through at least one aperture of the self-expanding stent.

6. The medical device assembly of any preceding claim, wherein the self-expanding stent is a drug-eluting stent.

7. A method of making a medical device assembly, the method comprising:
loading a self-expanding stent into an outer sheath, the self-expanding stent extending from a stent proximal end to a stent distal end and having a first length, the self-expanding stent expanding to an inner diameter of the outer sheath after loading;
loading a balloon catheter into the outer sheath, the balloon catheter including an inflatable balloon extending from a balloon proximal end to a balloon distal end and having a second length greater than the first length, the inflatable balloon being positioned such that the balloon proximal end lies proximal of the stent proximal end and the balloon distal end lies distal of the stent distal end; and
pressurizing the inflatable balloon such that the balloon proximal end contacts and at least partially abuts the stent proximal end.

8. The method of claim 7, further comprising heating the medical device assembly after pressurizing the inflatable balloon, for example wherein the outer sheath surrounds the inflatable balloon during heating.

9. The method of any of claims 7 to 8, wherein the balloon distal end surrounds the stent distal end.

10. The method of any of claims 7 to 9, wherein the balloon proximal end is in contact with an inner wall of the outer sheath after pressurizing.

11. The method of any of claims 7 to 10, wherein the stent comprises a plurality of apertures, for example wherein the inflatable balloon protrudes through at least one aperture of the self-expanding stent after pressurizing.

12. A method of delivering a stent, the method comprising:
delivering a medical device assembly in a compressed configuration to a site of treatment within a body lumen of a patient, the medical device assembly comprising:
a balloon catheter comprising an inflatable balloon, the inflatable balloon comprising a balloon proximal end and extending to a balloon distal end;
a self-expanding stent comprising a stent proximal end and extending to a stent distal end, the self-expanding stent being disposed over the inflatable balloon such that the balloon proximal end at least partially abuts the stent proximal end such that the inflatable balloon reduces longitudinal movement of the stent during deployment; and
an outer sheath surrounding the balloon catheter and self-expanding stent, such that the inflatable balloon acts to retain the stent against longitudinal movement during deployment;
withdrawing the outer sheath proximally to deploy the self-expanding stent to the site of treatment; and
inflating the inflatable balloon of the balloon catheter to seat the self-expanding stent against a wall of the body lumen at the treatment site.

13. The method of claim 12, wherein the balloon distal end at least partially abuts the stent distal end in the compressed configuration.

14. The method of claim 12 or 13, wherein the balloon proximal end contacts an inner wall of the outer sheath in the compressed configuration.

15. The method of any of claims 7 to 14, or the medical device assembly of any of claims 1 to 6 wherein the balloon catheter lacks a pusher element.
